# EUROPÄISCHE PATENTANMELDUNG

(11) **EP 3 441 037 A1**
(43) Veröffentlichungstag der Anmeldung: **13.02.2019**
(21) Anmeldenummer: 18186945.4
(22) Anmeldetag: 02.08.2018
(51) Int. Cl.: A61B 34/30, A61B 90/98, A61B 90/00

(54) **VERFAHREN ZUR ZULASSUNGSKONTROLLE EINES IN EINEM CHIRURGISCHEN ROBOTERSYSTEM EINZUSETZENDEN CHIRURGISCHEN INSTRUMENTS UND CHIRURGISCHES ROBOTERSYSTEM**

(30) Priorität: 11.08.2017 DE 102017118347
(71) Anmelder: avateramedical GmbH, 07745 Jena (DE)
(72) Erfinder: Seeber, Marcel, 07745 Jena (DE); Witt, Roberto, 07747 Jena (DE)
(74) Vertreter: Schaumburg und Partner Patentanwälte mbB

(57) **Zusammenfassung**

Ein chirurgisches Robotersystem (10) umfasst einen Manipulator (12) zur Bewegung und/oder Betätigung eines chirurgischen zur Verwendung bei einer einzigen Operation bestimmten Instrument (500), eine Steuereinheit (36) zum Steuern des Manipulators (12), eine Lese- und Schreibvorrichtung (160) zum Auslesen und Speichern von Daten eines Datenspeichers (494) des chirurgischen Instruments (500). Dabei ist die Lese- und Schreibvorrichtung (160) ausgebildet, im Zusammenhang mit einem Koppeln des chirurgischen Instruments (500) mit dem Robotersystem (10) aus dem Datenspeicher (494) erste Daten mit ersten Informationen zum chirurgischen Instrument (500) auszulesen und zur Steuereinheit (36) zu übertragen. Die Steuereinheit (36) überprüft ausgehend von den in den zweiten Daten enthaltenen zweiten Informationen, ob zumindest das eine Kompatibilitätskriterium mindestens eine in der Steuereinheit (36) voreingestellt gespeicherte Kompatibilitätsbedingung erfüllt. Die Steuereinheit (36) lässt das chirurgische Instrument (500) nur dann zur Benutzung durch das chirurgische Robotersystem (10) zu, wenn das Kompatibilitätskriterium die mindestens eine voreingestellt gespeicherte Kompatibilitätsbedingung erfüllt. Ein entsprechendes Verfahren zur Zulassungskontrolle stellt eine Verwendung eines chirurgischen Instruments (500) bei nur einer einzigen Operation sicher.

## Beschreibung

Die Erfindung betrifft ein Verfahren zur Zulassungskontrolle eines in einem chirurgischen Robotersystem einzusetzenden chirurgischen Instruments gemäß Anspruch 1 und ein chirurgisches Robotersystem gemäß dem Oberbegriff des Anspruchs 9.

Im Bereich der Chirurgie werden derartige Verfahren bzw. chirurgische Robotersysteme benutzt, um eine Zuverlässigkeit der Robotersysteme sicherstellen zu können und eine Fehlverwendung von Instrumenten im operativen Bereich zu vermeiden.

Aus dem Stand der Technik ist es bekannt, bei einer Kopplung eines chirurgischen Instruments mit einem Robotersystem zur Charakterisierung des zu koppelnden Instruments dem Robotersystem notwendige Daten bereitzustellen. So ist aus der EP 1146 830 B1 ein chirurgisches Robotersystem mit einem Prozessor und einer Werkzeughalterung und wenigstens einem chirurgischen Roboterwerkzeug bekannt. Das Roboterwerkzeug umfasst eine Welle mit einem proximalen Ende und einem distalen Ende, eine Schnittstelle an dem proximalen Ende der Welle, wobei die Schnittstelle an der Werkzeughalterung montiert werden kann. Ferner umfasst das Werkzeug einen Endeffektor, der mit dem distalen Ende der Welle gekoppelt ist, wobei der Endeffektor in wenigstens einem Freiheitsgrad relativ zu der Welle beweglich ist. Es ist ein Antriebssystem vorgesehen, das mit der Schnittstelle gekoppelt ist, um wenigstens ein mit dem wenigstens einen Freiheitsgrad des Endeffektors gekoppeltes Element anzutreiben. Eine Schaltungsanordnung ist mit der Schnittstelle gekoppelt, um für den Prozessor ein Signal bereitzustellen, das einen Werkzeugtyp des Werkzeugs und einen Bewegungsbereich des Endeffektors in dem wenigstens einen Freiheitsgrad angibt.

Der Prozessor ist dabei so ausgelegt, dass er den Werkzeugtyp und den Bewegungsbereich des Endeffektors von einem von der Werkzeughalterung gehaltenen Werkzeug anhand des Signals bestimmt, das von der Schaltungsanordnung, die mit der Schnittstelle des chirurgischen Roboterwerkzeugs gekoppelt ist, bereitgestellt wird. Das Signal gibt ferner die Werkzeuglebensdauer und/oder die kumulierte Werkzeugverwendung durch eine Messung eines kalendarischen Datums, einer Uhrzeit, einer Anzahl chirurgischer Prozeduren, einer Anzahl der Fälle, in denen das Werkzeug mit dem System gekoppelt wurde, oder einer Anzahl der Endeffektor-Betätigungen an.

Aus der EP 2 298 219 B1 ist ein chirurgisches Roboterwerkzeug zur Verwendung in einem chirurgischen Robotersystem bekannt, das einen Prozessor aufweist, der die Bewegung eines Werkzeughalters anweist. Das Werkzeug umfasst eine Sonde, die ein proximales Ende und ein distales Ende aufweist, einen chirurgischen Endeffektor, der benachbart zu dem distalen Ende der Sonde angeordnet ist, und eine Schnittstelle, die benachbart zu dem proximalen Ende der Sonde angeordnet ist. Dabei umfasst die Schnittstelle eine Schaltung, die an der Sonde angebracht ist und die ein Signal über die Schnittstelle an den Prozessor sendet. Das Werkzeug zeichnet sich dadurch aus, dass das Signal Werkzeugkalibrierungsversätze zwischen einer nominalen relativen Position des Abschnitts des Antriebssystems und des chirurgischen Endeffektors und einer gemessenen relativen Position des Abschnitts des Antriebssystems und des chirurgischen Endeffektors definiert.

Das Signal umfasst eindeutige Werkzeugkennungsdaten oder zeigt mindestens eine Werkzeugstandzeit und einen kumulativen Werkzeugeinsatz durch eine Messung eines Kalenderdatums, einer Uhrzeit, einer Anzahl der chirurgischen Eingriffe, einer Anzahl von Kopplungsvorgängen, bei denen das Werkzeug mit dem System gekoppelt wurde, oder einer Anzahl der Betätigungen des Endeffektors an.

EP 2 363 091 B1 offenbart ein chirurgisches Robotersystem mit einem chirurgischen Werkzeug, einem Robotermanipulator mit einem Werkzeughalter, an den das chirurgische Werkzeug lösbar gekoppelt werden kann. Der Robotermanipulator ist dafür ausgelegt, das chirurgische Werkzeug so zu manipulieren, dass sich das chirurgische Werkzeug bewegt, und mit einem Prozessor zum Steuern des Robotermanipulators, sodass er die Bewegung des chirurgischen Werkzeugs steuert. Das chirurgische Werkzeug weist eine Sonde mit einem proximalen und einem distalen Ende, einen chirurgischen Endeffektor, der benachbart zu dem distalen Ende der Sonde angeordnet ist, eine Schnittstelle, die benachbart zu dem proximalen Ende der Sonde angeordnet ist, wobei die Schnittstelle lösbar mit dem Werkzeughalter gekoppelt werden kann, und eine Schaltung auf, die an der Sonde montiert ist.

Die Schaltung definiert dabei ein Signal zur Übertragung an den Prozessor, um die Kompatibilität des Werkzeugs mit dem System anzuzeigen, wobei das Signal eindeutige Identifikationsdaten und eine verschlüsselte Verifikationsdatenfolge umfasst, die aus den eindeutigen Identifikationsdaten gemäß einem Algorithmus berechnet wird. Dabei ist der Prozessor konfiguriert für ein Empfangen des Signals von dem Werkzeug, wenn es mit dem Werkzeughalter gekoppelt ist, ein Manipulieren der eindeutigen Identifikationsdaten mit dem Algorithmus, um Bestätigungsdaten zu erzeugen, ein Vergleichen der Bestätigungsdaten mit der verschlüsselten Verifikationsdatenfolge, um die Kompatibilität des Werkzeugs mit dem chirurgischen Robotersystem zu verifizieren und Erlauben, dass das chirurgische Robotersystem das Werkzeug manipuliert, wenn die Bestätigungsdaten der verschlüsselten Verifikationsdatenfolge entsprechen.

Darüber hinaus besteht insbesondere hinsichtlich von Instrumenten, die nur zu einer Verwendung bei einer einzigen Operation bestimmt sind, sogenannten Einmalinstrumenten, ein Bedarf, die Einhaltung von Sterilitätserfordernissen an chirurgische Instrumente sicherzustellen.

Der Erfindung liegt daher die Aufgabe zugrunde, ein Verfahren und ein chirurgisches Robotersystem anzugeben, durch die eine unerlaubte Verwendung eines chirurgischen Instruments einschließlich einer unbeabsichtigten Mehrfachverwendung eines solchen Instruments ausgeschlossen werden kann. Insbesondere soll eine Mehrfach-Verwendung eines Einmalinstruments, welches bereits in einer medizinischen Operation verwendet wurde, ausgeschlossen werden. Ebenso soll eine Verwendung eines chirurgischen Instruments ausgeschlossen werden, wenn mindestens ein vorgebbarer Parameter des Instruments einen geforderten Gerätewert nicht aufweist bzw. den zulässigen Gerätewert nicht einhält.

Die Erfindung löst diese Aufgabe durch ein Verfahren mit den Merkmalen des Anspruchs 1 und durch ein chirurgisches Robotersystem mit den Merkmalen des Anspruchs 9.

Gemäß einem ersten Aspekt sieht die Erfindung ein Verfahren zur Zulassungskontrolle eines in einem chirurgischen Robotersystem einzusetzenden chirurgischen Instruments mit einem Datenspeicher vor, in dem erste Daten mit ersten Informationen zum chirurgischen Instrument und zweite Daten mit Informationen zu mindestens einem Kompatibilitätskriterium des chirurgischen Robotersystems hinsichtlich des chirurgischen Instruments gespeichert sind. Bei diesem Verfahren wird das chirurgische Instrument mit dem chirurgischen Robotersystem gekoppelt, es werden die ersten Daten aus dem Datenspeicher ausgelesen und mithilfe der in den ersten Daten enthaltenen ersten Informationen wird geprüft, ob das chirurgische Instrument zum Einsatz in dem chirurgischen Robotersystem geeignet ist. In dem Fall, dass beim Prüfen eine Eignung des chirurgischen Instruments zum Einsatz in dem chirurgischen Robotersystem ermittelt wird, werden die zweiten Daten aus dem Datenspeicher ausgelesen und es wird mit Hilfe der in den zweiten Daten enthaltenen zweiten Informationen geprüft, ob zumindest das eine Kompatibilitätskriterium mindestens eine im chirurgischen Robotersystem voreingestellt gespeicherte Kompatibilitätsbedingung erfüllt. Weiter wird das chirurgische Instrument bei dem Verfahren nur dann zur Benutzung durch das chirurgische Robotersystem zugelassen, wenn das Kompatibilitätskriterium die mindestens eine voreingestellt gespeicherte Kompatibilitätsbedingung erfüllt. Hierdurch kann sowohl sichergestellt werden, dass das Instrument für das Robotersystem überhaupt geeignet, d.h. kompatibel, ist als auch, dass das Instrument nur bei einer einzigen Operation eingesetzt werden kann, so dass dasselbe Instrument nicht bei einer Operation eines anderen Patienten eingesetzt werden kann, weder versehentlich noch vorsätzlich.

Gemäß einem zweiten Aspekt sieht die Erfindung ein chirurgisches Robotersystem mit wenigstens einem Manipulator zur Bewegung und/oder Betätigung eines chirurgischen Instruments, welches insbesondere zur Verwendung bei einer einzigen Operation bestimmt ist, mit einer Steuereinheit zum Steuern des Manipulators, mit einer Lese- und Schreibvorrichtung zum Auslesen und Speichern von Daten eines Datenspeichers des chirurgischen Instruments vor. Dabei ist die Lese- und Schreibvorrichtung ausgebildet, im Zusammenhang mit einem Koppeln des chirurgischen Instruments mit dem Robotersystem aus dem Datenspeicher erste Daten mit ersten Informationen zum chirurgischen Instrument auszulesen und zur Steuereinheit zu übertragen.

Das chirurgische Robotersystem zeichnet sich dadurch aus, dass der Datenspeicher des chirurgischen Instruments zweite Daten mit Informationen zu mindestens einem Kompatibilitätskriterium des chirurgischen Robotersystems hinsichtlich des chirurgischen Instruments enthält, dass die Lese- und Schreibvorrichtung ausgebildet ist, im Zusammenhang mit dem Koppeln des chirurgischen Instruments mit dem Robotersystem aus dem Datenspeicher zumindest die ersten Daten auszulesen und zur Steuereinheit zu übertragen, und dass die Steuereinheit ausgebildet ist, mithilfe der in den ersten Daten enthaltenen ersten Informationen zu prüfen, ob das chirurgische Instrument zum Einsatz in dem chirurgischen Robotersystem geeignet ist. Weiter zeichnet sich das chirurgische Robotersystem dadurch aus, dass die Lese- und Schreibvorrichtung die zweiten Daten aus dem Datenspeicher ausliest und zur Steuereinheit überträgt, wenn die Steuereinheit beim Prüfen eine Eignung des chirurgischen Instruments zum Einsatz in dem chirurgischen Robotersystem festgestellt hat. Zusätzlich zeichnet sich das chirurgische Robotersystem dadurch aus, dass die Steuereinheit ausgebildet ist, ausgehend von den in den zweiten Daten enthaltenen zweiten Informationen zu prüfen, ob zumindest das eine Kompatibilitätskriterium mindestens eine in der Steuereinheit voreingestellt gespeicherte Kompatibilitätsbedingung erfüllt, und dass die Steuereinheit das chirurgische Instrument nur dann zur Benutzung durch das chirurgische Robotersystem zulässt, wenn das Kompatibilitätskriterium die mindestens eine voreingestellt gespeicherte Kompatibilitätsbedingung erfüllt.

Das Robotersystem bzw. eine Steuereinheit des Robotersystems kann im Wesentlichen die Verfahrensschritte des Verfahrens nach Anspruch 1 ausführen, um sowohl sicherzustellen, dass das Instrument für das Robotersystem überhaupt geeignet, d.h. kompatibel, ist, als auch, dass das Instrument nur bei einer einzigen Operation eingesetzt werden kann, so dass dasselbe Instrument nicht bei einer Operation eines anderen Patienten eingesetzt werden kann, weder versehentlich noch vorsätzlich.

Somit haben das Verfahren gemäß dem ersten Aspekt und das Robotersystem gemäß dem zweiten Aspekt den Vorteil, bei chirurgischen Operationen bzw. Eingriffen eine Kompatibilität von darin einzusetzenden chirurgischen Instrumenteneinheiten bzw. Instrumenten sicherzustellen. Ferner stellt die Erfindung sicher, dass chirurgische Instrumenteneinheiten bzw. Instrumente sowohl konform mit den produktrelevanten Spezifikationen als auch konform mit den erforderlichen Sterilitätskriterien eingesetzt werden können.

Das Robotersystem gemäß dem zweiten Aspekt kann in gleicher Weise weitergebildet werden, wie das Verfahren nach Anspruch 1, insbesondere mit den Merkmalen der abhängigen Aussprüche. Auch das Verfahren nach Anspruch 1 kann mit Merkmalen des Robotersystems, insbesondere mit Merkmalen der abhängigen Ansprüche weitergebildet werden.

Bei einer Ausführungsform umfassen die zweiten Daten einen Informationsplatzhalter zum Hinzufügen einer Operationsidentifikationskennzeichnung. Der Informationsplatzhalter kann in einfacher Ausführung als reservierter Speicher ausgebildet sein. Als Kompatibilitätsbedingung wird geprüft, ob der Informationsplatzhalter in den zweiten Daten vorhanden ist. Weiter umfasst das Kompatibilitätskriterium eine von dem Robotersystem vorgebbare Operationsidentifikationskennzeichnung, eine System-Operationsidentifikationskennzeichnung, zur eindeutigen Identifizierung einer einzigen Operation, bei der das chirurgische Instrument eingesetzt werden soll. Die vom Robotersystem vorgegebene System-Operationsidentifikationskennzeichnung wird im Bereich des Informationsplatzhalters der zweiten Daten als Instrument-Operationsidentifikationskennzeichnung gespeichert.

Bei einer anderen Ausführungsform stellt der Informationsplatzhalter selbst ein vorläufiges Kompatibilitätskriterium auf oder enthält ein vorläufiges Kompatibilitätskriterium. Als Kompatibilitätsbedingung kann dann das Vorhandensein des vorläufigen Kompatibilitätskriteriums geprüft werden.

Ferner kann als Kompatibilitätsbedingung die Übereinstimmung der durch das chirurgische Robotersystem vorgegebenen System-Operationsidentifikationskennzeichnung mit der in den zweiten Daten als Kompatibilitätskriterium enthaltenen Instrument-Operationsidentifikationskennzeichnung geprüft werden.

Weiterhin können die ersten Daten eine das chirurgische Instrument zumindest hinsichtlich seiner Funktion identifizierende Seriennummer, ein Produktionsdatum und/oder eine maximale Lagerungszeit des chirurgischen Instruments oder ein Ablaufdatum des chirurgischen Instruments enthalten, wobei überprüft wird, ob die maximale Lagerungszeit oder das Ablaufdatum überschritten worden ist.

Weiterhin können die ersten oder die zweiten Daten oder beide Daten eine Prüfsumme zu deren Verifikation enthalten. Mithilfe der Prüfsumme kann überprüft werden, ob die Daten unversehrt übertragen, korrekt ausgelesen und nicht manipuliert worden sind.

Auch können die ersten oder die zweiten Daten oder beide Daten verschlüsselt gespeichert und/oder übertragen werden. Eine Verschlüsselung dient der Datensicherheit. Auch ist es denkbar, dass die ersten und/oder zweiten Daten mit einem Schreibschutz versehen werden, wobei der Schreibschutz der zweiten Daten vorzugsweise nach dem Speichern der System-Operationsidentifikationskennzeichnung aktiviert wird und wobei der Schreibschutz nach dem Aktivieren vorzugsweise nicht mehr deaktivierbar ist. Durch den Schreibschutz wird eine Datenmanipulation wirkungsvoll verhindert. Dadurch kann sichergestellt werden, dass in dem Datenspeicher des chirurgischen Instruments fortwährend die passenden Daten zu dessen Beschreibung und Identifizierung vorgehalten werden. Die Lese- und Schreibvorrichtung ist in dieser Ausführungsform des erfindungsgemäßen chirurgischen Robotersystems zum Entschlüsseln und Verschlüsseln der ersten und/oder der zweiten Daten ausgebildet.

Bei einer weiteren Ausführung umfassen die ersten Daten Informationen mit einem vorläufigen Kompatibilitätskriterium. Diese können durch einen entsprechenden Speicherplatz vorgebende binäre Codes realisiert sein. Denkbar ist aber auch, dass die Informationen mit dem vorläufigen Kompatibilitätskriterium zumindest in ihrer Datenstruktur den Informationen mit dem Kompatibilitätskriterium angepasst sind bzw. diesen gleichen.

In einer vorteilhaften Ausführungsform speichert die Steuereinheit die voreingestellt gespeicherte System-Operationsidentifikationskennzeichnung als Instrument-Operationsidentifikationskennzeichnung in den zweiten Daten, wenn bei der Überprüfung in den zweiten Daten keine Informationen zu einer konkreten Operation ermittelt werden.

Alternativ oder zusätzlich kann die Steuereinheit eines erfindungsgemäßen chirurgischen Robotersystems die voreingestellt gespeicherte System-Operationsidentifikationskennzeichnung in den zweiten Daten als Instrument-Operationsidentifikationskennzeichnung speichern, wenn bei der Überprüfung der Daten in dem Datenspeicher lediglich ein Informationsplatzhalter ermittelt wird.

So kann sichergestellt werden, dass die chirurgische Instrumenteneinheit bzw. das chirurgische Instrument nur für eine einzige, eindeutig identifizierte, chirurgische Operation zum Einsatz kommt.

Im Fall einer zur Verwendung bei einer einzigen Operation bestimmten chirurgischen Instrumenteneinheit oder eines Einmalinstruments bedeutet eine Speicherung der vom Robotersystem erzeugten System-Operationsidentifikationskennzeichnung als Instrument-Operationsidentifikationskennzeichnung auf dem Datenspeicher der Instrumenteneinheit bzw. des Instruments, dass die Instrumenteneinheit bzw. das Instrument bei jeder weiteren, durch eine abweichende System-Operationsidentifikationskennzeichnung ausgewiesenen chirurgischen Operation verworfen wird. Die System-Operationsidentifikationskennzeichnung kann vorteilhaft aus alphanumerischen Zeichen bestehen. Denkbar ist als System-Operationsidentifikationskennzeichnung aber auch eine Zahlenfolge.

Die chirurgische Instrumenteneinheit bzw. das chirurgische Instrument wird derart durch das übergeordnete Robotersystem bzw. die Steuereinheit somit derart markiert, dass sie bzw. es in einer anderen Operation nicht mit dem Robotersystem gekoppelt und genutzt werden kann.

Bei einer Zulässigkeitsprüfung der chirurgischen Instrumenteneinheit bzw. des chirurgischen Instruments kann ein Ergebnis der Prüfung sein, die chirurgische Instrumenteneinheit bzw. das chirurgische Instrument mit der System-Operationsidentifikationskennzeichnung einer konkreten Operation zu markieren. Das heißt die chirurgische Instrumenteneinheit bzw. das chirurgische Instrument kann von der Steuereinheit bzw. dem übergeordneten Robotersystem während eines Operationsvorganges mit dem Robotersystem verbunden, genutzt, wieder entkoppelt und erneut verbunden werden.

Der Datenspeicher kann mithilfe einer Lese- und Schreibvorrichtung des Robotersystems ausgelesen werden. Eine Auswertung der Daten erfolgt durch eine Steuereinheit des Robotersystems. Ein Ergebnis der Auswertung kann darin liegen, dass die chirurgische Instrumenteneinheit bzw. das chirurgische Instrument noch nicht in einer Operation eingesetzt wurde, oder dass eine Lagerungszeit kürzer als ein maximal vorgegebener Lagerungszeitraum ist, sodass eine Verwendung der chirurgischen Instrumenteneinheit bzw. des chirurgischen Instruments von dem Robotersystem bzw. der Steuereinheit zugelassen werden kann.

Die ersten oder die zweiten Daten oder beide Daten können einen Informationsplatzhalter enthalten. Auch kann der Informationsplatzhalter aus anderen, in den ersten und/oder zweiten Daten enthaltenen weiteren Instrumentendaten mithilfe eines Algorithmus erzeugt werden.

Weiterhin können die zweiten Daten neben dem Informationsplatzhalter auch die chirurgische Instrumenteneinheit bzw. das chirurgische Instrument beschreibende Informationen wie solche, das Instrument hinsichtlich seiner Funktion, wie Schere, Greifer, Dissektor und dgl., beschreibende Informationen, ein Herstellungsdatum und/oder eine maximale Lagerungszeit enthalten. Mit Vorteil kann dann so hinsichtlich der Daten eine Redundanz erreicht werden.

In einer Weiterbildung sind die ersten Daten mit einem Schreibschutz versehen, in einem nur einmal beschreibbaren Speicher oder in einem Nur-Lese-Speicher (ROM) gespeichert.

In einer bevorzugten Ausführungsform ist die Steuereinheit ausgebildet, die ersten Daten auszulesen und/oder zu empfangen sowie die ersten Daten zu überprüfen.

Eine weitere Ausführungsform zeichnet sich dadurch aus, dass eine Prüfvorrichtung zum Prüfen des chirurgischen Instruments die ersten und/oder zweiten Daten in dem Datenspeicher der chirurgischen Instrumenteneinheit speichert.

So kann etwa bei einer am Ende eines Herstellungsvorgangs der chirurgischen Instrumenteneinheit bzw. des chirurgischen Instruments stattfindenden Endprüfung in einer entsprechenden Prüfvorrichtung eine für die chirurgische Instrumenteneinheit und/oder das chirurgische Instrument eindeutige Seriennummer erzeugt werden, die zusammen mit weiteren Informationen, wie etwa einem Produktionsdatum, einer maximalen Lagerungszeit oder einem Ablaufdatum, in den ersten Daten hinterlegt wird. Bei einer Speicherung werden die ersten Daten mit einer Prüfsumme versehen, verschlüsselt und in einem ihnen zugeordneten Datenbereich des Datenspeichers in der chirurgischen Instrumenteneinheit gespeichert. Nach dem Vorgang des Speicherns der ersten Daten wird der Datenbereich für die ersten Daten in der Regel mit einem Schreibschutz versehen.

Ebenso können in den zweiten Daten des Datenspeichers Informationsplatzhalter für die System-Operationsidentifikationskennzeichnung gespeichert werden. Dabei kann es sich etwa um eine binäre Zahlenfolge aus Nullen oder Einsen handeln. Bevorzugt kann in dem Informationsplatzhalter oder auch als Informationsplatzhalter ein vorläufiges Kompatibilitätskriterium hinterlegt werden. Ein solches vorläufiges Kompatibilitätskriterium enthält noch nicht dieselbe Information wie die von dem Robotersystem vorgebbare oder darin voreingestellt gespeicherte System-Operationsidentifikationskennzeichnung, weist aber zumindest den gleichen Aufbau der Datenstruktur auf.

Es können etwa im Rahmen der Endprüfung neben den in dem Datenspeicher enthaltenen Informationsplatzhaltern weiter bevorzugt die Seriennummer und weitere Informationen, wie etwa das Produktionsdatum, die maximale Lagerungszeit oder das Ablaufdatum der chirurgischen Instrumenteneinheit und/oder des chirurgischen Instruments, als zweite Daten gespeichert werden. Dann kann eine Redundanz hinsichtlich der die chirurgische Instrumenteneinheit bzw. das chirurgische Instrument beschreibenden Daten erreicht werden. Eine Datenstörung oder eine Fehlauslesung etwa der ersten Daten könnte durch die in den zweiten Daten enthaltenen Informationen ignoriert oder korrigiert werden.

Bei dem Verfahren bzw. bei dem Robotersystem kann der Informationsplatzhalter auch in den ersten Daten enthalten sein. Dann kann das vorläufige Kompatibilitätskriterium vorzugsweise zur Authentifizierung der chirurgischen Instrumenteneinheit bzw. des chirurgischen Instruments bei der Steuereinheit des Robotersystems verwendet werden. Die Steuereinheit kann dazu das vorläufige Kompatibilitätskriterium beispielsweise aus den weiteren, die chirurgische Instrumenteneinheit bzw. das chirurgische Instrument beschreibenden Daten mithilfe eines Algorithmus berechnen.

Bei einer weiteren Ausführungsform ist die Lese- und Schreibvorrichtung ausgebildet, die die chirurgische Instrumenteneinheit bzw. das chirurgische Instrument beschreibenden ersten Daten aus dem Datenspeicher bei dem Koppeln der chirurgischen Instrumenteneinheit bzw. des chirurgischen Instruments mit dem Robotersystem auszulesen. Dazu kann ein Auslesen über eine Nahbereichs-Sende-Empfängereinrichtung, wie z.B. einer RFID-Lese- und Schreibeinheit, erfolgen, sodass die Daten bereits ausgelesen werden können, wenn die chirurgische Instrumenteneinheit bzw. das chirurgische Instrument im Rahmen von Operationsvorbereitungen in die Nähe des Robotersystems gebracht worden ist. Ebenso kann das Auslesen der Daten aber auch erst nach Herstellung einer drahtgebundenen Kommunikationsverbindung mit dem Robotersystem bzw. der Steuereinheit erfolgen.

In einer bevorzugten Ausführungsform enthalten die ersten Daten ein Herstellungsdatum und eine maximale Lagerungszeit oder ein Ablaufdatum. Die Steuereinheit ist dann ausgebildet, zu überprüfen, ob die maximale Lagerungszeit oder das Ablaufdatum abgelaufen ist.

Sowohl bei dem erfindungsgemäßen Verfahren als auch bei dem erfindungsgemäßen Robotersystem kann ein Auslesen bzw. Schreiben von Daten aus dem bzw. in den Datenspeicher der chirurgischen Instrumenteneinheit oder des chirurgischen Instruments über einen Funkfrequenzidentifikationschip (RFID-Chip bzw. RFID-Tag) erfolgen. Denkbar ist aber auch ein optisches Auslesen und Speichern der Daten im Rahmen einer optischen Datenübertragung. Ferner kann bei einer leitungsgebundenen Variante ein Flashspeicher zum Einsatz kommen. Zumindest die ersten Daten könnten auch in einem Code, wie einem Quick Response (QR)-Code, bereitgestellt werden.

Um sowohl das chirurgische Instrument als auch die chirurgische Instrumenteneinheit bei nur einer einzigen Operation einsetzen zu können, können beide einen Datenspeicher aufweisen, in dem jeweils eine eine Operation eindeutig identifizierende Kennung, die System-Operationsidentifikationskennzeichnung S-OID, als Instrument-Operationsidentifikationskennzeichnung I-OID gespeichert, vorzugsweise schreibgeschützt gespeichert wird. Dadurch kann nach Beendigung der durch die System- bzw. Instrument-Operationsidentifikationskennzeichnung identifizierten Operation entweder die chirurgische Instrumenteneinheit oder das chirurgische Instrument von einer weiteren Nutzung ausgeschlossen werden.

Weitere Merkmale und Vorteile ergeben sich aus der folgenden Beschreibung, die die Erfindung anhand von Ausführungsbeispielen im Zusammenhang mit den beigefügten, in unterschiedlichen Maßstäben gehaltenen Figuren näher erläutert. Die dargestellten Elemente sind dabei teilweise vereinfacht dargestellt.

Es zeigen:
- Fig. 1: eine schematische Darstellung eines Robotersystems zur robotergestützten Chirurgie mit einem Manipulator, der vier Manipulatorarme hat, mit denen jeweils eine Sterileinheit einer Instrumenteneinheit verbindbar ist;
- Fig. 2: eine perspektivische Darstellung eines Abschnitts eines Manipulatorarms mit einer Koppeleinheit zum Koppeln des Manipulatorarms mit einer eine Sterileinheit umfassenden Instrumenteneinheit mit einer mit der Koppeleinheit gekoppelten Sterilschleuse und mit einer mit der Sterilschleuse gekoppelten Sterileinheit der Instrumenteneinheit;
- Fig. 3: eine schematische Darstellung der Koppeleinheit des Manipulatorarms mit ausgeblendeter Gehäuseoberseite;
- Fig. 4: eine Draufsicht auf die Instrumenteneinheit mit entfernter Bodenplatte;
- Fig. 5: ein Ablaufdiagramm zur Veranschaulichung des erfindungsgemäßen Verfahrens bzw. der Funktion des Robotersystems und
- Fig. 6: ein weiteres Ablaufdiagramm zur Veranschaulichung eines Verfahrens zur Zulassungskontrolle eines in dem chirurgischen Robotersystem einzusetzenden chirurgischen Instruments sowie zur Veranschaulichung der Funktion des Robotersystems.

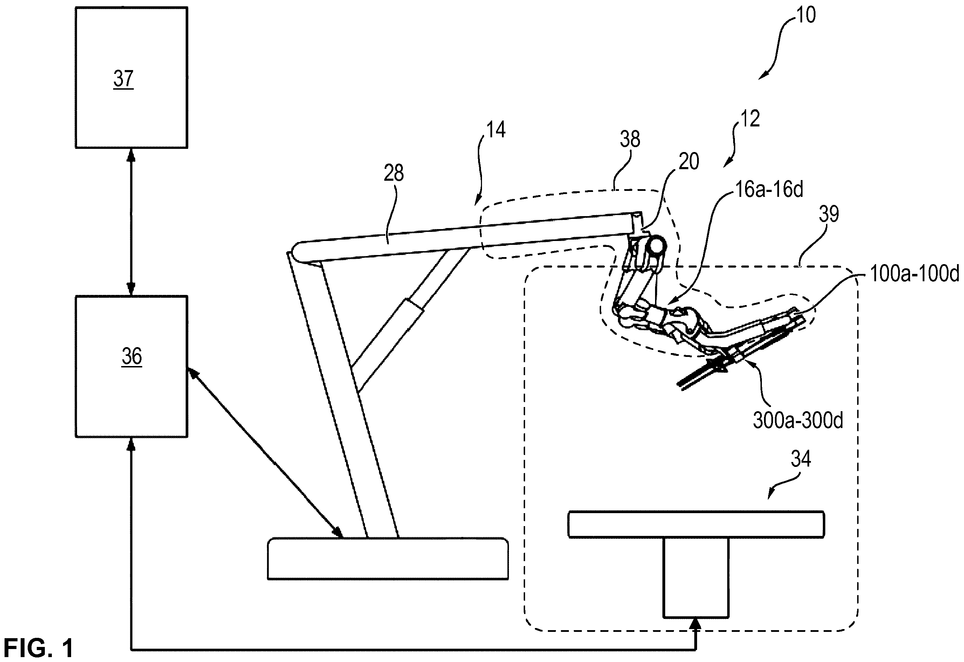

Fig. 1 zeigt eine schematische Darstellung des chirurgischen Robotersystems 10 zum Einsatz in der robotergestützten Chirurgie. Das Robotersystem 10 hat einen Manipulator 12, der ein Stativ 14 und vorzugsweise vier Manipulatorarme 16a bis 16d hat. Jeder Manipulatorarm 16a bis 16d ist mit einer sterilen Instrumenteneinheit 300a bis 300d über eine Koppeleinheit 100a bis 100d des Manipulatorarms 16a bis 16d verbunden.

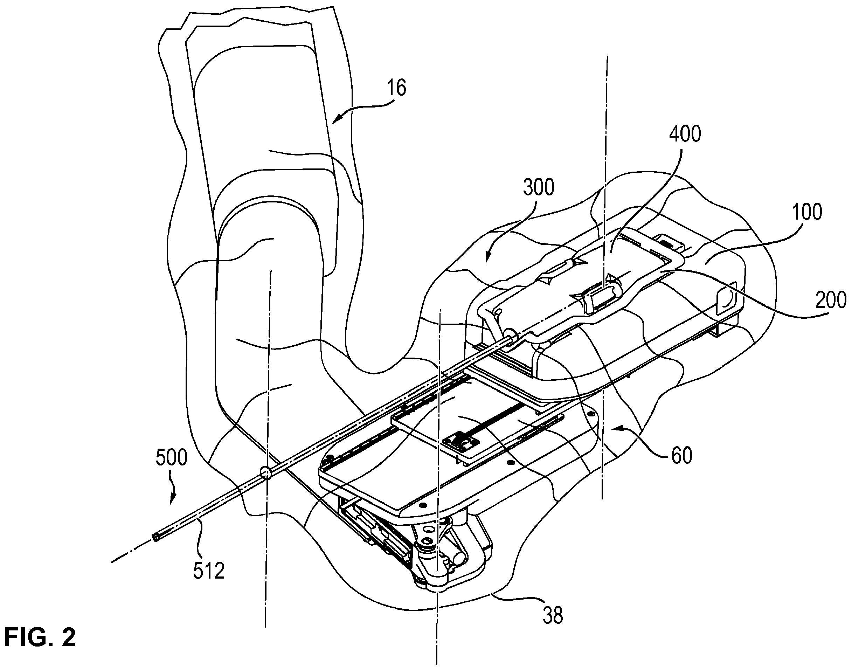

Die Instrumenteneinheit 300a bis 300d ist steril und umfasst neben einer in Figur 2 gezeigten Sterileinheit 400 zum Koppeln der Instrumenteneinheit 300a bis 300d mit der Koppeleinheit 100a bis 100d des Manipulatorarms 16a bis 16d ein chirurgisches Instrument 500, insbesondere mit einem Endeffektor, wobei der Endeffektor mithilfe der Koppeleinheit 100a bis 100d des Manipulatorarms 16a bis 16d bewegt und/oder betätigt werden kann. Alternativ zum chirurgischen Instrument 500 kann die Instrumenteneinheit 300a bis 300d auch ein optisches Instrument, insbesondere ein Endoskop, und/oder ein medizinisches Gerät, insbesondere zum Applizieren eines Medikaments, zum Abgeben einer Spülflüssigkeit und/oder zum Absaugen von Spülflüssigkeit und/oder Sekret, umfassen.

Der Manipulator 12 wird mithilfe einer Steuereinheit 36 gesteuert. Die Steuereinheit 36 ist über eine Daten- und/oder Steuerleitung mit einer Ein- und Ausgabeeinheit 37 verbunden, die insbesondere ein Bild des Operationsfelds an eine Bedienperson in Echtzeit mithilfe mindestens einer Anzeigeeinheit der Ein- und Ausgabeeinheit 37 ausgibt. Die Bedienperson macht Bedieneingaben, durch die die Instrumenteneinheiten 300a bis 300d während der Operation eines Patienten positioniert und betätigt werden. Die Ein- und Ausgabeeinheit 37 dient somit als Mensch-Maschinen-Schnittstelle.

Die Steuereinheit 36 ist ferner über eine Steuer- und/oder Datenverbindung mit einer nicht dargestellten Steuereinheit des Operationstischs 34 verbunden. Über diese Steuer- und/oder Datenverbindung wird sichergestellt, dass die Lage der Patientenlagerfläche oder von Segmenten der Patientenlagerfläche des Operationstischs 34 nur dann verändert werden kann, wenn dies aufgrund der Positionierung der Instrumenteneinheiten 300a bis 300d für einen zu operierenden Patienten gefahrlos möglich ist.

Der Operationstisch 34 sowie die Instrumenteneinheiten 300a bis 300d sind in einem sterilen Operationsbereich 39 angeordnet. Die Manipulatorarme 16a bis 16d und das Stativ 14 sind nicht steril. Die in dem sterilen Operationsbereich 39 ragenden Bereiche des Manipulators 12, d. h. die Manipulatorarme 16a bis 16d , der Stativkopf 20 und ein Teil des Stativarms 28 , sind in einer mithilfe der Strichlinie angedeuteten sterilen flexiblen Hülle 38 , wie einer Sterilfolie, steril verpackt, sodass sie gefahrlos im sterilen Operationsbereich 39 angeordnet werden können. Die Ein- und Ausgabeeinheit 37 ist außerhalb des sterilen Bereichs 39 angeordnet und muss deshalb nicht steril verpackt werden.

Bei einer Vielzahl von Operationen müssen die Instrumenteneinheiten 300a bis 300d während der Operation aufgrund des Operationsverlaufs mehrfach gewechselt werden. Somit muss zwischen dem Manipulatorarm 16a bis 16d und der Instrumenteneinheit 300a bis 300d eine sterile Schnittstelle derart vorgesehen werden, dass die nicht sterilen Teile der Koppeleinheit des Manipulatorarms 16a bis 16d auch nach dem Trennen der Instrumenteneinheit 300a bis 300d steril abgedeckt sind.

Darüber hinaus müssen durch einen Kontakt der sterilen Elemente der Koppeleinheit des Manipulatorarms 16a bis 16d kontaminierte Elemente der Instrumenteneinheit 300a bis 300d nach dem Trennen der Instrumenteneinheit 300a bis 300d vom Manipulatorarm 16a bis 16d steril abgedeckt werden, damit die Instrumenteneinheit 300a bis 300d im sterilen Bereich 39 abgelegt werden kann, ohne weitere Elemente im sterilen Bereich 39 zu kontaminieren. Dazu wird eine Sterilschleuse 200 zwischen der Koppeleinheit 100a bis 100d des Manipulatorarms 16a bis 16d und der Instrumenteneinheit 300a bis 300d vorgesehen, die mindestens eine Schleusenklappe hat, die geschlossen ist, wenn keine Instrumenteneinheit 300a bis 300d mit der Sterilschleuse 200 verbunden ist, sodass dann die nicht sterile Koppeleinheit 100a bis 100d vom sterilen Bereich 39 mithilfe der flexiblen sterilen Hülle 38 und der in diese integrierten Sterilschleuse vom sterilen Bereich 39 abgeschirmt ist.

In Fig. 2 ist ein Teil eines Manipulatorarms 16a bis 16d mit einer Koppeleinheit 100a bis 100d sowie eine mit der Koppeleinheit 100a bis 100d über eine Sterilschleuse 200 verbundene Instrumenteneinheit 300a bis 300d gezeigt. Da die nachfolgende Erläuterung für die Manipulatorarme 16a bis 16d und die Instrumenteneinheiten 300a bis 300d gleich ist, wird auf diese unter den Bezugszeichen 16 und 300 Bezug genommen. Entsprechend zeigt Fig. 2 eine perspektivische Darstellung eines Abschnitts eines Manipulatorarms 16 mit einer Koppeleinheit 100 zum Koppeln des Manipulatorarms 16 mit der eine Sterileinheit 400 und ein Instrument 500 umfassenden Instrumenteneinheit 300. Hierzu ist die Koppeleinheit 100 mit einer in eine sterile Hülle 38 integrierten Sterilschleuse 200 verbunden. Die Sterilschleuse 200 ist sowohl mit der Koppeleinheit 100 als auch mit einer Sterileinheit 400 koppelbar und wieder trennbar. In Fig. 2 ist die Sterilschleuse 200 sowohl mit der Koppeleinheit 100 als auch mit der Sterileinheit 400 gekoppelt dargestellt. Die Koppeleinheit 100 ist am distalen Ende einer Teleskopanordnung 60 angeordnet.

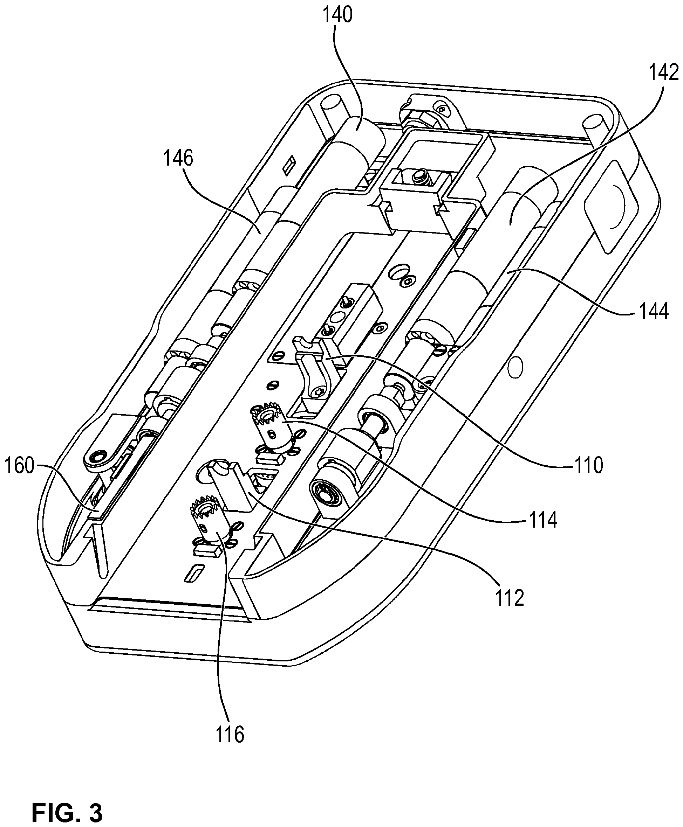

Fig. 3 zeigt eine Koppeleinheit 100 mit ausgeblendeter Gehäuseoberseite. Die Koppeleinheit 100 hat insgesamt vier Antriebsmotoren 140 bis 146 , die jeweils als Gleichstrommotoren mit Tachometer ausgeführt sind, sodass die Steuereinheit 36 jederzeit über den Drehwinkel des jeweiligen Antriebsmotors 140 bis 146 Kenntnis hat und dies bei der weiteren Ansteuerung berücksichtigen kann. Der erste Antriebsmotor 140 ist über ein erstes Linearkoppelgetriebe mit dem ersten translatorischen Antriebselement 110 gekoppelt, das bei einer Aktivierung des Antriebsmotors 140 durch die Steuereinheit 36 eine translatorische Antriebsbewegung ausführt. Der zweite Antriebsmotor 142 ist über ein zweites Linearkoppelgetriebe mit dem zweiten translatorischen Antriebselement 112 gekoppelt, sodass bei einer Antriebsbewegung des zweiten Antriebsmotors 142 das zweite translatorische Antriebselement 112 eine translatorische Antriebsbewegung ausführt. Der dritte Antriebsmotor 144 ist über eine erste Getriebestufe mit dem ersten rotatorischen Antriebselement 114 gekoppelt, sodass bei einer Antriebsbewegung des dritten Antriebsmotors 144 das erste rotatorische Antriebselement 114 gedreht wird. Der vierte Antriebsmotor 146 ist über eine zweite Getriebestufe mit dem zweiten rotatorischen Antriebselement 116 gekoppelt, sodass dieses bei einer Antriebsbewegung des vierten Antriebsmotors 146 eine Rotationsbewegung ausführt.

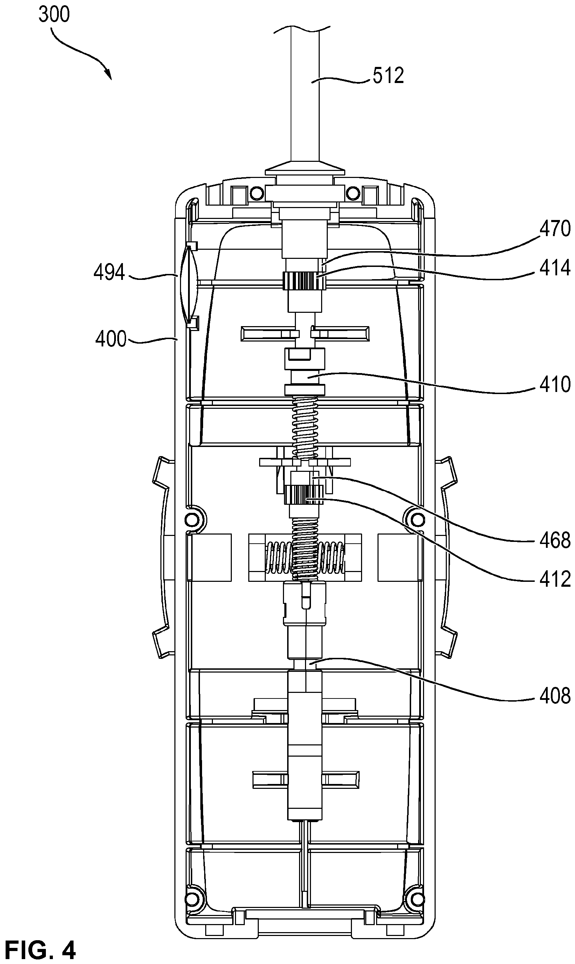

Fig. 4 zeigt die Instrumenteneinheit 300, bei der eine nicht dargestellte Bodenplatte der Sterileinheit 400 entfernt worden ist. Mehrere Elemente 408 bis 414 werden mithilfe der Antriebselemente 110 bis 116 der Koppeleinheit 100 angetrieben. Das als Zahnrad ausgebildete zweite rotatorisch angetriebene Element 414 ist mit dem äußeren Instrumentenschaft 512 und einem zweiten Winkelgeber 470 für die Detektion der Schaftdrehung des äußeren Instrumentenschafts 512 drehfest verbunden.

Das zweite translatorisch angetriebene Element 410 ist mit einem ersten inneren Instrumentenschaft verbunden, sodass bei einer translatorischen Bewegung des zweiten translatorisch angetriebenen Elements 410 der erste innere Instrumentenschaft translatorisch bewegt wird.

Das erste rotatorisch angetriebene Element 412 ist drehfest mit einem ersten Winkelgeber 468 und einem zweiten inneren Instrumentenschaft drehfest verbunden. Der zweite innere Instrumentenschaft dient zur Endeffektordrehung unabhängig von dem Drehwinkel des äußeren Instrumentenschafts 512. Der erste innere Instrumentenschaft dient zur Abwinkelung des Endeffektors. Ausgehend von dem Robotersystem 10 wird nachfolgend die Funktion einer Zulassungskontrolle der bei einer Operation eines Patienten eingesetzten Instrumenteneinheiten 300 beschrieben.

Die in Fig. 4 gezeigte chirurgische Instrumenteneinheit 300 ist mit einem Datenspeicher 494 ausgestattet, der vorzugsweise als Funkfrequenzidentifikationschip (RFID-Chip) ausgeführt ist. Der Datenspeicher 494 wird während einer Herstellung der chirurgischen Instrumenteneinheit 300 bei einer Prüfung der chirurgischen Instrumenteneinheit 300 auf einem nicht gezeigten Prüfstand mit Daten beschrieben. Sofern eine Prüfung auf Funktionsfähigkeit positiv verlaufen ist, werden eine Seriennummer, ein Produktionsdatum, eine Chargennummer und/oder eine Produktionsnummer erzeugt und Daten mit diesen Informationen in dem Datenspeicher 494 gespeichert. Die gespeicherten Daten können ferner Informationen über den Instrumententyp, die maximale Lagerungszeit und/oder eine Information, dass das chirurgische Instrument noch nicht bei einer medizinischen Operation eingesetzt worden ist, umfassen.

In einer vorzugsweise im Rahmen eines Herstellungsprozesses stattfindenden Endprüfung der chirurgischen Instrumenteneinheit 300 bzw. des chirurgischen Instruments 500 wird für jede Instrumenteneinheit 300 bzw. für jedes Instrument 500 eine eindeutige Seriennummer SNo erzeugt und diese zusammen mit wenigstens einer weiteren Information, wie einem Produktionslos Lot oder einer maximalen Lagerungszeit SL, in einem ersten Informationsblock IB1 zusammengefasst. Der erste Informationsblock IB1 wird mit einer Prüfsumme versehen, verschlüsselt und in einem dem Informationsblock IB1 zugeordneten Datenbereich des Datenspeichers 494 in der chirurgischen Instrumenteneinheit 300 gespeichert. Der Datenbereich für den Informationsblock IB1 wird nach dem Beschreiben mit einem Schreibschutz versehen.

Zum Einsatz der Instrumenteneinheit 300 bzw. des chirurgischen Instruments 500 in Verbindung mit dem chirurgischen Robotersystem 10 liest eine Lese- und Schreibvorrichtung 160 Daten aus dem Datenspeicher 494 aus. Die gelesenen Daten werden zur Steuereinheit 36 des Robotersystems 10 übertragen und von dieser ausgewertet. Die Auswertung soll sicherstellen, dass die chirurgische Instrumenteneinheit 300 bzw. das chirurgische Instrument 500 noch nicht in einer anderen chirurgischen Operation verwendet wurde und dass die Lagerungszeit innerhalb einer zugelassenen Grenze liegt. Entsprechend kann eine Verwendung der chirurgischen Instrumenteneinheit 300 bzw. des chirurgischen Instruments 500 durch die Steuereinheit 36 des Robotersystems 10 zugelassen werden. Die Steuereinheit 36 speichert in dem Datenspeicher 494 der chirurgischen Instrumenteneinheit 300 eine Instrument-Operationsidentifikationskennzeichnung, durch die sichergestellt wird, dass die Instrumenteneinheit 300 während ein und derselben Operation beliebig oft mit dem Robotersystem 10 gekoppelt, genutzt und entkoppelt werden kann, jedoch nicht bei einer weiteren Operation. Vorzugsweise erzeugt das Robotersystem 10 eine System-Operationsidentifikationskennzeichnung, die vorzugsweise einzigartig ist, nur für eine einzige Operation gültig ist und die während der Operation auf dem Instrument als Instrument-Operationsidentifikationskennzeichnung dient.

Insbesondere werden in dem Datenspeicher 494 mindestens zwei Informationsblöcke IB1, IB2 verschlüsselt gespeichert. Der erste Informationsblock IB1 umfasst zum Beispiel eine Prüfsumme CHK des ersten Informationsblocks IB1, eine Seriennummer SNo, ein Produktionslos Lot und eine maximale Lagerungszeit SL.

| | | | | |
|---|---|---|---|---|
| CHK | SNo | Lot | SL | *(IB1)* |

Der Informationsblock IB2 kann mindestens eine eindeutige Operationsidentifikationskennzeichnung (OID) oder einen Informationsplatzhalter OIDP enthalten und/oder die Prüfsumme CHK des zweiten Informationsblocks IB2, die Seriennummer SNo, das Produktionslos Lot, die maximale Lagerungszeit SL und/oder weitere Informationen. Der Informationsplatzhalter OIDP kann je nach Beschaffenheit selbst ein vorläufiges Kompatibilitätskriterium darstellen oder enthalten.

| | | | | | |
|---|---|---|---|---|---|
| CHK | SNo | Lot | SL | I-OID/OIDP | *(IB2)* |

Der im Datenspeicher 494 gespeicherte Informationsplatzhalter OIDP kann auch eine eindeutige Operationsidentifikationskennzeichnung OID, insbesondere eine Instrument-Operationsidentifikationskennzeichnung I-OID enthalten. Die durch das Robotersystem 10 erzeugte und in der Steuereinheit 36 gespeicherte eindeutige Operationsidentifikationskennzeichnung wird auch als System-Operationsidentifikationskennzeichnung S-OID bezeichnet.

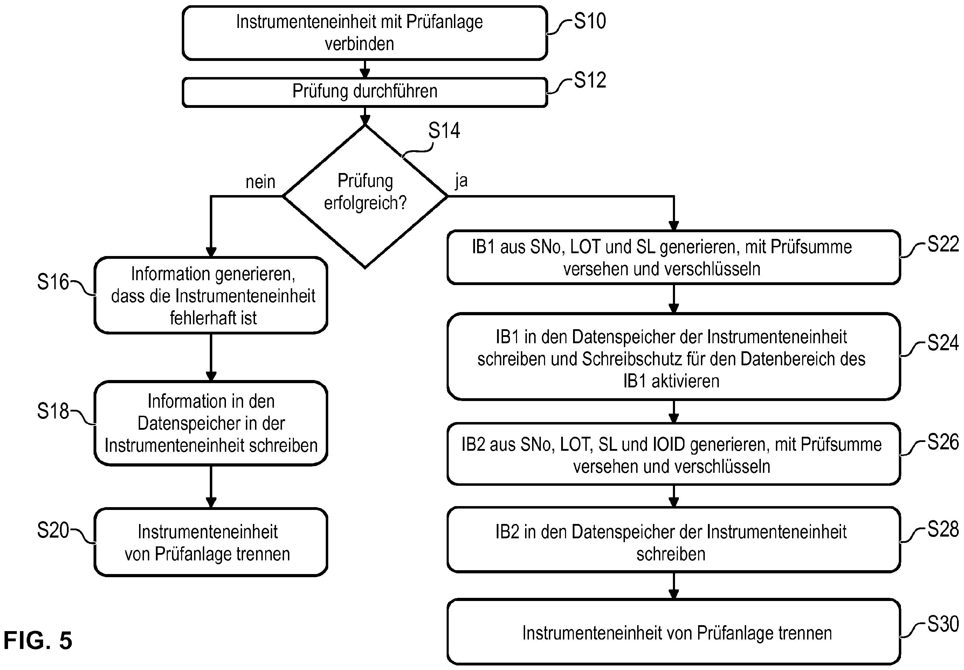

Fig. 5 zeigt ein Ablaufdiagramm zur Veranschaulichung des erfindungsgemäßen Verfahrens bzw. der Funktion des Robotersystems 10. Im Schritt S10 wird die chirurgische Instrumenteneinheit 300 mit einer nicht gezeigten Prüfvorrichtung verbunden, die im Schritt S12 eine Prüfung der Instrumenteneinheit 300 auf ihre Einsatztauglichkeit durchführt. Dabei prüft die Prüfvorrichtung im Schritt S14 die chirurgische Instrumenteneinheit 300 bzw. das chirurgische Instrument 500 daraufhin, ob sie bzw. es funktionstauglich ist, ob alle konstruktiven Elemente korrekt angebracht sind und ob die Instrumenteneinheit 300 bzw. das Instrument 500 den für einen klinischen Einsatz erforderlichen Reinheits- bzw. Sterilitätsbedingungen entspricht. Das impliziert eine Überprüfung, dass es sich bei der einzusetzenden chirurgischen Instrumenteneinheit 300 bzw. dem Instrument 500 um eine bzw. ein noch nicht bei einer anderen Operation eingesetzte Instrumenteneinheit 300 bzw. eingesetztes Instrument 500 handelt.

Sofern sich bei der Prüfung gemäß Schritt S14 ergibt, dass die chirurgische Instrumenteneinheit 300 bzw. das chirurgische Instrument 500 nicht über die notwendige Funktionstauglichkeit und/oder nicht über die notwendige Sterilität verfügt, wird die chirurgische Instrumenteneinheit 300 bzw. das Instrument 500 im Schritt S16 als fehlerhaft eingestuft. Eine entsprechende Information wird dann im Schritt S18 in dem Datenspeicher 494 der Instrumenteneinheit 300 gespeichert. Entsprechend wird die chirurgische Instrumenteneinheit 300 bzw. das Instrument 500 nach einer Trennung von der Prüfanlage im Schritt S20 ausgesondert.

Ist das Ergebnis der Prüfung im Schritt S14 der chirurgischen Instrumenteneinheit 300 bzw. des chirurgischen Instruments 500 durch die Prüfvorrichtung positiv, werden im Schritt S22 Daten mit dem Informationsblock IB1 aus der Seriennummer SNo, dem Produktionslos Lot und der maximalen Lagerungszeit SL erzeugt, mit einer Prüfsumme versehen und verschlüsselt. Im Schritt S24 werden anschließend Daten mit dem Informationsblock IB1 in den Datenspeicher 494 der chirurgischen Instrumenteneinheit 300 geschrieben und ein Schreibschutz für den Datenbereich, in dem sich die Daten mit dem Informationsblock IB1 befinden, aktiviert. Weiter werden im Schritt S26 Daten mit dem Informationsblock IB2 aus dem Informationsplatzhalter OIDP erzeugt, mit einer Prüfsumme versehen und verschlüsselt. Ferner kann der zweite Informationsblock IB2 zusätzliche Informationen wie die Seriennummer SNo, die Produktionsnummer Lot und die maximale Lagerungszeit SL enthalten. Die Daten mit dem Informationsblock IB2 werden dann im Schritt S28 in den Datenspeicher 494 der chirurgischen Instrumenteneinheit 300 geschrieben. Anschließend wird die chirurgische Instrumenteneinheit 300 im Schritt S30 von der Prüfvorrichtung getrennt.

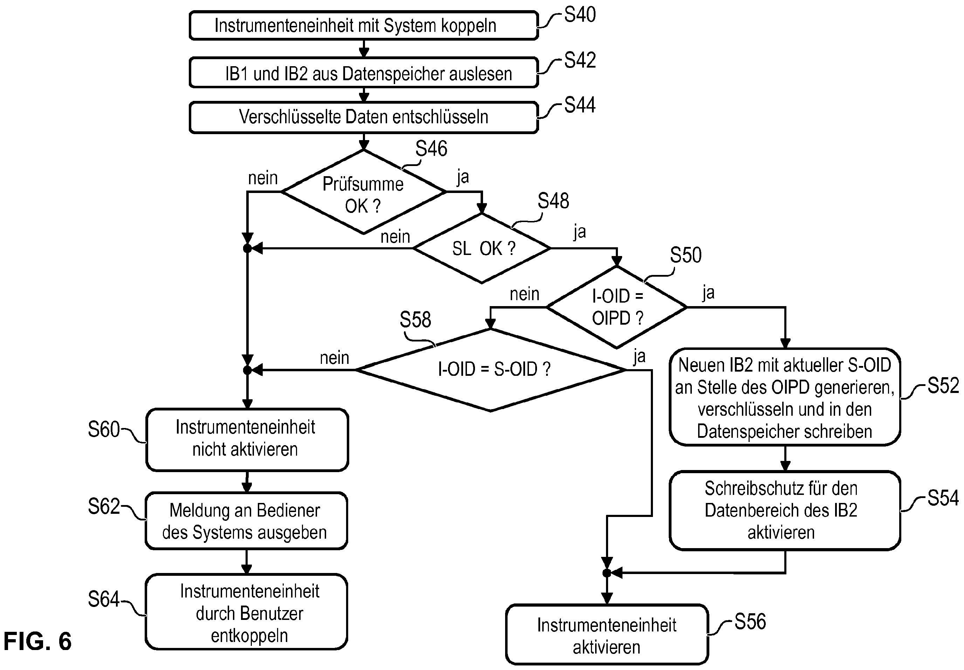

Fig. 6 zeigt ein weiteres Ablaufdiagramm zur Veranschaulichung eines Verfahrens zur Zulassungskontrolle eines in dem chirurgischen Robotersystem 10 einzusetzenden chirurgischen Instruments sowie zur Veranschaulichung der Funktion des Robotersystems 10.

Eine wie anhand der Fig. 5 beschrieben getestete chirurgische Instrumenteneinheit 300 wird beim Ablauf nach Fig. 6 im Schritt S40 mit dem Robotersystem 10 gekoppelt. Erste Daten mit dem Informationsblock IB1 und zweite Daten mit dem Informationsblock IB2 werden im Schritt S42 aus dem Datenspeicher 494 ausgelesen und die verschlüsselten ersten und zweiten Daten werden im Schritt S44 entschlüsselt. Ergibt eine Prüfung im Schritt S46, dass eine Prüfsumme einem erwarteten Wert entspricht, so wird im Schritt S48 weiter überprüft, ob die maximale Lagerungszeit SL noch nicht überschritten ist. Wenn diese Prüfung im Schritt S48 ergibt, dass die maximale Lagerungszeit SL noch nicht abgelaufen ist, wird im Schritt S50 weiter geprüft, ob es sich bei der in den Daten enthaltenen Instrument-Operationsidentifikationskennzeichnung I-OID um einen Informationsplatzhalter OIDP handelt. Sofern dieses der Fall ist, bedeutet das, dass die chirurgische Instrumenteneinheit 300 noch nicht bei einer Operation oder einem chirurgischen Eingriff eingesetzt wurde. Daraufhin werden im Schritt S52 Daten mit einem neuen Informationsblock IB2 mit einer aktuellen, von dem Robotersystem 10, d. h. der Steuereinheit 36, erzeugten eindeutigen System-Operationsidentifikationskennzeichnung S-OID erzeugt, verschlüsselt und in den Datenspeicher 494 der chirurgischen Instrumenteneinheit 300 als zweite Daten geschrieben. Nachfolgend wird im Schritt S54 ein Schreibschutz für den Datenbereich des Informationsblocks IB2 aktiviert. Somit ist im neu erzeugten Informationsblock IB2 der Informationsplatzhalter OIDP durch die System-Operationsidentifikationskennzeichnung S-OID ersetzt worden und dient nach dem Speichern des neu erzeugten Informationsblocks IB2 als Instrument-Operationsidentifikationskennzeichnung I-OID. Anschließend wird die chirurgische Instrumenteneinheit 300 im Schritt S56 für eine Benutzung durch das Robotersystem 10 aktiviert.

Wird bei der Prüfung im Schritt S50 jedoch festgestellt, dass die in den Daten enthaltene Instrument-Operationsidentifikationskennzeichnung I-OID kein Informationsplatzhalter OIDP ist, wird anschließend im Schritt S58 weiter überprüft, ob die Instrument-Operationsidentifikationskennzeichnung I-OID mit der System-Operationsidentifikationskennzeichnung S-OID übereinstimmt.

In dem Fall, dass die Instrument-Operationsidentifikationskennzeichnung I-OID mit der System-Operationsidentifikationskennzeichnung S-OID übereinstimmt, wird die chirurgische Instrumenteneinheit 300 bzw. das Instrument 500 zu einer Benutzung durch das Robotersystem 10 im Schritt S56 aktiviert. Dieser Fall tritt beispielsweise bei einem Einsatz der chirurgischen Instrumenteneinheit 300 bzw. des chirurgischen Instruments 500 in einem Robotersystem 10 bei einer Operation auf, wenn während einer Operation die Instrumenteneinheiten 300 gewechselt werden müssen, also eine chirurgische Instrumenteneinheit 300 bzw. ein chirurgisches Instrument 500 nicht permanent mit dem Robotersystem verbunden bzw. gekoppelt bleibt, sondern für einen oder mehrere Zeiträume von dem Robotersystem 10 getrennt und wieder mit ihm verbunden wird. In einem solchen Fall ist in dem Datenspeicher 494 der chirurgischen Instrumenteneinheit 300 bereits die für die aktuelle Operation individuell erzeugte System-Operationsidentifikationskennzeichnung S-OID in dem zweiten Datenblock IB2 des Datenspeichers 494 als Instrument-Operationsidentifikationskennzeichnung I-OID gespeichert. Dieser Fall kann insbesondere dadurch eintreten, dass alle für eine geplante Operation vorgesehenen Instrumenteneinheiten bei der Operationsvorbereitung mit den Manipulatorarmen 16 verbunden werden.

Stimmt die im Datenspeicher 494 gespeicherte eindeutige Instrument-Operationsidentifikationskennzeichnung I-OID nicht mit der durch das Robotersystem 10 erzeugten eindeutigen Operationsidentifikationskennzeichnung S-OID überein, so wird die chirurgische Instrumenteneinheit 300 im Schritt S60 nicht zur Benutzung durch das Robotersystem 10 aktiviert. Weiterhin wird im Schritt S62 eine Meldung an einen Bediener bzw. Benutzer des Robotersystems 10 ausgegeben. Die chirurgische Instrumenteneinheit 300 wird dann im Schritt S64 durch den Bediener bzw. Benutzer von der Koppeleinheit 100 des Robotersystems 10 entkoppelt. Ferner wird eine Ansteuerung des Manipulatorarms 16, mit dem diese Instrumenteneinheit 300 verbunden ist, durch die Steuereinheit 36 verhindert.

Die Schritte S60 bis S64 werden auch durchlaufen, wenn bei der Prüfung nach Schritt S46 eine nicht-korrekte Prüfsumme ermittelt wird oder bei der Prüfung im Schritt S48 festgestellt wird, dass die maximale Lagerungszeit SL bereits abgelaufen ist.

Bei einer erstmaligen Benutzung der chirurgischen Instrumenteneinheit 300 in einem chirurgischen Robotersystem 10 werden nach einem Koppeln der chirurgischen Instrumenteneinheit 300 erste Daten mit dem ersten Informationsblock IB1 und zweite Daten mit dem zweiten Informationsblock IB2 aus dem Datenspeicher 494 ausgelesen und entschlüsselt. Ferner wird anhand einer Prüfsumme die Gültigkeit der Daten überprüft. Bei einer positiven Prüfung wird der Inhalt überprüft, ob redundante Geräteangaben der beiden Informationsblöcke IB1 und IB2 identisch sind. Weiter wird überprüft, ob die maximale Lagerungszeit SL noch nicht abgelaufen ist, und ob die chirurgische Instrumenteneinheit 300 bzw. das Instrument 500 noch nicht bei einer Operation benutzt worden ist.

Ergibt diese Überprüfung, dass die Instrumenteneinheit 300 noch nicht bei einer Operation benutzt worden ist, kann noch keine durch ein Robotersystem 10 erzeugte System-Operationsidentifikationskennzeichnung S-OID in dem entsprechenden Bereich des Informationsblocks IB2 als Instrument-Identifikationskennzeichnung I-OID gespeichert worden sein. Die zweiten Daten mit dem Informationsblock IB2 werden dann unter Benutzung einer aktuell gültigen, von dem Robotersystem 10 erzeugten eindeutigen Operationsidentifikationskennzeichnung S-OID neu erzeugt, mit einer Prüfsumme versehen, verschlüsselt und im Datenspeicher 494 gespeichert. Ferner wird ein Schreibschutz für den Datenbereich der Daten des Informationsblocks IB2 im Datenspeicher 494 aktiviert. Dadurch wird erreicht, dass der Datenbereich mit den zweiten Daten des Informationsblocks IB2 nur noch lesbar ist. Anschließend wird die gekoppelte Instrumenteneinheit 300 für eine aktuell anstehende Operation aktiviert.

Im Fall einer wiederholten Benutzung einer so konfigurierten chirurgischen Instrumenteneinheit 300 in einem chirurgischen Robotersystem 10 werden nach einem Koppeln der chirurgischen Instrumenteneinheit 300 die ersten Daten mit dem Informationsblock IB1 und die zweiten Daten mit dem Informationsblock IB2 aus dem Datenspeicher 494 ausgelesen und entschlüsselt. Es wird anhand einer Prüfsumme überprüft, ob die Daten gültig sind. Ist die Prüfung positiv, wird eine Überprüfung durchgeführt, ob die einander entsprechenden Daten der beiden Informationsblöcke IB1 und IB2 identisch sind.

Weiter wird überprüft, ob eine maximale Lagerungszeit SL noch nicht abgelaufen ist, und ob die Instrumenteneinheit 300 bzw. das Instrument 500 noch nicht benutzt worden ist. In dem Fall, dass die chirurgische Instrumenteneinheit 300 bzw. das Instrument 500 bereits benutzt worden ist, wird die in der Instrumenteneinheit 300 gespeicherte eindeutige Instrument-Operationsidentifikationskennzeichnung I-OID mit der von dem Robotersystem 10 erzeugten eindeutigen System-Operationsidentifikationskennzeichnung S-OID verglichen.

Stimmen System-Operationsidentifikationskennzeichnung S-OID und Instrument-Operationsidentifikationskennzeichnung I-OID überein, wird die chirurgische Instrumenteneinheit 300 zur Benutzung von der Steuereinheit 36 zugelassen. In dem Fall, dass die auf dem Robotersystem 10 erzeugte eindeutige System-Operationsidentifikationskennzeichnung S-OID mit der in der chirurgischen Instrumenteneinheit 300 gespeicherten eindeutigen Instrument-Operationsidentifikationskennzeichnung I-OID nicht übereinstimmt, wird die chirurgische Instrumenteneinheit 300 nicht zur Benutzung zugelassen und der Anwender darüber informiert. Dadurch wird verhindert, dass eine chirurgische Instrumenteneinheit 300 bzw. ein chirurgisches Instrument 500, die bzw. das bereits bei einer durch die Instrument-Operationsidentifikationskennzeichnung I-OID gekennzeichneten Operation eingesetzt wurde, erneut für eine andere Operation eingesetzt wird.

Der Datenspeicher 494 kann ein Datenspeicher eines RFID-Chips sein, der mit Hilfe einer RFID-Schreib- und Leseeinheit beschreibbar und lesbar ist. Bei anderen Ausführungsformen kann die Instrumenteneinheit 300 andere Speicher umfassen, die als Datenspeicher 494 dienen, insbesondere einen über WLAN oder Bluetooth auslesbaren und/oder beschreibbaren Speicher.

### Bezugszeichenliste

- 10: System
- 12: Manipulator
- 14: Stativ
- 16, 16a bis 16d: Manipulatorarm
- 20: Stativkopf
- 28: Stativarme
- 34: Operationstisch
- 36: Steuereinheit
- 37: Ein- und Ausgabeeinheit
- 38: sterile Hülle
- 39: steriler Operationsbereich
- 60: Segment
- 100, 100a bis 100d: Koppeleinheit
- 110: erstes translatorisches Antriebselement/Linearhubgabel
- 112: zweites translatorisches Antriebselement/Linearhubgabel
- 114: erstes rotatorisches Antriebselement/Antriebsritzel
- 116: zweites rotatorisches Antriebselement/Antriebsritzel
- 140, 142, 144, 146: Antriebsmotor
- 160: RFID-Lese- und Schreibvorrichtung
- 200: Sterilschleuse
- 300, 300a bis 300d: Instrumenteneinheit
- 400: Sterileinheit
- 408: erstes translatorisches angetriebenes Element
- 410: zweites translatorisches angetriebenes Element
- 412: erstes rotatorisches angetriebenes Element
- 414: zweites rotatorisches angetriebenes Element
- 468: erster Winkelgeber
- 470: zweiter Winkelgeber
- 494: RFID-Datenspeicher
- 500: Instrument
- 512: äußerer Instrumentenschaft
- S10 bis S64: Verfahrensschritte
- IB1: erster Informationsblock
- IB2: zweiter Informationsblock
- OID: Operationsidentifikationskennzeichnung
- S-OID: System-Operationsidentifikationskennzeichnung
- I-OID: Instrument-Operationsidentifikationskennzeichnung
- OIDP: Operationsidentifikationskennzeichnungsplatzhalter

## Patentansprüche

1. Verfahren zur Zulassungskontrolle eines in einem chirurgischen Robotersystem (10) einzusetzenden chirurgischen Instruments (500) mit einem Datenspeicher (494), in dem erste Daten mit ersten Informationen zum chirurgischen Instrument (500) und zweite Daten mit Informationen zu mindestens einem Kompatibilitätskriterium des chirurgischen Robotersystems (10) hinsichtlich des chirurgischen Instruments (500) gespeichert sind,
bei dem das chirurgische Instrument (500) mit dem chirurgischen Robotersystem (10) gekoppelt wird (S 40),
bei dem die ersten Daten aus dem Datenspeicher (494) ausgelesen werden (S 42) und mithilfe der in den ersten Daten enthaltenen ersten Informationen geprüft wird, ob das chirurgische Instrument (500) zum Einsatz in dem chirurgischen Robotersystem (10) geeignet ist (S 48),
bei dem in dem Fall, dass beim Prüfen eine Eignung des chirurgischen Instruments (500) zum Einsatz in dem chirurgischen Robotersystem (10) festgestellt wird, die zweiten Daten aus dem Datenspeicher (494) ausgelesen werden und mit Hilfe der in den zweiten Daten enthaltenen zweiten Informationen geprüft wird, ob zumindest das eine Kompatibilitätskriterium mindestens eine im chirurgischen Robotersystem (10) voreingestellt gespeicherte Kompatibilitätsbedingung erfüllt, und
bei dem das chirurgische Instrument (500) nur dann zur Benutzung durch das chirurgische Robotersystem (10) zugelassen wird, wenn das Kompatibilitätskriterium die mindestens eine voreingestellt gespeicherte Kompatibilitätsbedingung erfüllt.

2. Verfahren nach Anspruch 1, **dadurch gekennzeichnet, dass** die zweiten Daten einen Informationsplatzhalter (OIDP) zum Hinzufügen einer System-Operationsidentifikationskennzeichnung (S-OID) umfassen, dass als Kompatibilitätsbedingung geprüft wird, ob der Informationsplatzhalter (OIDP) in den zweiten Daten vorhanden ist, dass als Kompatibilitätskriterium eine System-Operationsidentifikationskennzeichnung (S-OID) zur eindeutigen Identifizierung einer einzigen Operation, bei der das chirurgische Instrument ( 500) eingesetzt werden soll, von dem Robotersystem (10) vorgegeben wird, und dass die vorgegebene System-Operationsidentifikationskennzeichnung (S-OID) im Bereich des Informationsplatzhalters (OIDP) der zweiten Daten als eine Instrument-Operationsidentifikationskennzeichnung (I-OID) gespeichert wird.

3. Verfahren nach Anspruch 2, **dadurch gekennzeichnet, dass** der Informationsplatzhalter (OIDP) selbst ein vorläufiges Kompatibilitätskriterium darstellt und/oder enthält und dass als Kompatibilitätsbedingung das Vorhandensein des vorläufigen Kompatibilitätskriteriums geprüft wird.

4. Verfahren nach Anspruch 2 oder 3, **dadurch gekennzeichnet, dass** als Kompatibilitätsbedingung die Übereinstimmung der durch das chirurgische Robotersystem (10) vorgegebenen System-Operationsidentifikationskennzeichnung (S-OID) und der in den zweiten Daten als Kompatibilitätskriterium enthaltenen Instrument-Operationsidentifikationskennzeichnung (I-OID) geprüft wird.

5. Verfahren nach einem der Ansprüche 1 bis 4, **dadurch gekennzeichnet, dass** die ersten Daten eine das chirurgische Instrument (500) zumindest hinsichtlich seiner Funktion identifizierende Seriennummer (SNo), ein Produktionsdatum (Lot) und/oder eine maximale Lagerungszeit (SL) des chirurgischen Instruments (500) oder ein Ablaufdatum des chirurgischen Instruments (500) enthalten und dass als Kompatibilitätsbedingung überprüft wird, ob die maximale Lagerungszeit (SL) (S 48) oder das Ablaufdatum überschritten worden ist.

6. Verfahren nach einem der Ansprüche 1 bis 5, **dadurch gekennzeichnet, dass** die ersten und/oder die zweiten Daten eine Prüfsumme zu deren Verifikation enthalten.

7. Verfahren nach einem der Ansprüche 1 bis 6, **dadurch gekennzeichnet, dass** die ersten Daten und/oder die zweiten Daten mit einem Schreibschutz versehen werden (S 54), wobei der Schreibschutz der zweiten Daten vorzugsweise nach dem Speichern der System-Operationsidentifikationskennzeichnung (S-OID) aktiviert wird, wobei der Schreibschutz nach dem Aktivieren nicht mehr deaktivierbar ist.

8. Verfahren nach einem der Ansprüche 1 bis 7, **dadurch gekennzeichnet, dass** die ersten Daten Informationen mit einem vorläufigen Kompatibilitätskriterium umfassen.

9. Chirurgisches Robotersystem (10) mit wenigstens einem Manipulator (12) zur Bewegung und/oder Betätigung eines chirurgischen Instruments (500), welches insbesondere zur Verwendung bei einer einzigen Operation bestimmt ist,
mit einer Steuereinheit (36) zum Steuern des Manipulators (12),
mit einer Lese- und Schreibvorrichtung (160) zum Auslesen und Speichern von Daten eines Datenspeichers (494) des chirurgischen Instruments (500),
wobei die Lese- und Schreibvorrichtung (160) ausgebildet ist, im Zusammenhang mit einem Koppeln des chirurgischen Instruments (500) mit dem Robotersystem (10) aus dem Datenspeicher (494) erste Daten mit ersten Informationen zum chirurgischen Instrument (500) auszulesen und zur Steuereinheit (36) zu übertragen,
**dadurch gekennzeichnet, dass** der Datenspeicher (494) des chirurgischen Instruments (500) zweite Daten mit Informationen zu mindestens einem Kompatibilitätskriterium des chirurgischen Robotersystems (10) hinsichtlich des chirurgischen Instruments (500) enthält,
dass die Lese- und Schreibvorrichtung (160) ausgebildet ist, im Zusammenhang mit dem Koppeln des chirurgischen Instruments (500) mit dem Robotersystem (10) aus dem Datenspeicher (494) zumindest die ersten Daten auszulesen und zur Steuereinheit (36) zu übertragen,
dass die Steuereinheit (36) ausgebildet ist, mithilfe der in den ersten Daten enthaltenen ersten Informationen zu prüfen, ob das chirurgische Instrument (500) zum Einsatz in dem chirurgischen Robotersystem (10) geeignet ist (S 48),
dass die Lese- und Schreibvorrichtung (160) die zweiten Daten aus dem Datenspeicher ausliest und zur Steuereinheit (36) überträgt, wenn die Steuereinheit (36) beim Prüfen eine Eignung des chirurgischen Instruments (500) zum Einsatz in dem chirurgischen Robotersystem (10) festgestellt hat,
dass die Steuereinheit (36) ausgebildet ist, ausgehend von den in den zweiten Daten enthaltenen zweiten Informationen zu prüfen, ob zumindest das eine Kompatibilitätskriterium mindestens eine in der Steuereinheit (36) voreingestellt gespeicherte Kompatibilitätsbedingung erfüllt, und
dass die Steuereinheit (36) das chirurgische Instrument (500) nur dann zur Benutzung durch das chirurgische Robotersystem (10) zulässt, wenn das Kompatibilitätskriterium die mindestens eine voreingestellt gespeicherte Kompatibilitätsbedingung erfüllt.

10. Chirurgisches Robotersystem nach Anspruch 9, **dadurch gekennzeichnet, dass** die zweiten Daten einen Informationsplatzhalter (OIDP) zum Hinzufügen einer System-Operationsidentifikationskennzeichnung (S-OID) umfassen, dass die Steuereinheit (36) als Kompatibilitätsbedingung das Vorhandensein des Informationsplatzhalters (OIDP) in den zweiten Daten prüft, dass die Steuereinheit (36) eine System-Operationsidentifikationskennzeichnung (S-OID) zur eindeutigen Identifizierung einer einzigen Operation, bei der das chirurgische Instrument (500) eingesetzt werden soll, vorgibt und dass die Lese- und Schreibvorrichtung (160) die vorgegebene System-Operationsidentifikationskennzeichnung (S-OID) im Bereich des Informationsplatzhalters (OIDP) der zweiten Daten als Instrument-Operationsidentifikationskennzeichnung (I-OID) speichert.

11. Chirurgisches Robotersystem nach Anspruch 10, **dadurch gekennzeichnet, dass** der Informationsplatzhalter (OIDP) ein vorläufiges Kompatibilitätskriterium darstellt und/oder enthält und dass als Kompatibilitätsbedingung das Vorhandensein des vorläufigen Kompatibilitätskriteriums geprüft wird.

12. Chirurgisches Robotersystem nach einem der Ansprüche 9 bis 11, **dadurch gekennzeichnet, dass** die Steuereinheit (36) als Kompatibilitätsbedingung die Übereinstimmung der durch das chirurgische Robotersystem (10) vorgegebenen System-Operationsidentifikationskennzeichnung (S-OID) mit der in den zweiten Daten als Kompatibilitätskriterium enthaltenen Instrument-Operationsidentifikationskennzeichnung (I-OID) prüft.

13. Chirurgisches Robotersystem nach einem der Ansprüche 9 bis 12, **dadurch gekennzeichnet, dass** eine Prüfvorrichtung zum Prüfen des chirurgischen Instruments (500) vor einer Operation die ersten und/oder zweiten Daten in dem Datenspeicher (494) der chirurgischen Instrumenteneinheit (500) speichert.

14. Chirurgisches Robotersystem nach einem der Ansprüche 9 bis 13, **dadurch gekennzeichnet, dass** die Lese- und Schreibvorrichtung (160) ausgebildet ist, die ersten Daten aus dem Datenspeicher (494) bei dem Koppeln des chirurgischen Instruments (500) mit dem Robotersystem (10) auszulesen.

15. Chirurgisches Robotersystem nach einem der Ansprüche 9 bis 14, **dadurch gekennzeichnet, dass** die ersten Daten ein Herstellungsdatum (Lot) und eine maximale Lagerungszeit (SL) enthalten und/oder dass die ersten Daten ein Ablaufdatum enthalten und dass die Steuereinheit (36) ausgebildet ist, zu überprüfen, ob die maximale Lagerungszeit (SL) oder das Ablaufdatum abgelaufen ist.
